# EUROPÄISCHE PATENTSCHRIFT

(11) **EP 1 197 431 B1**
(45) Veröffentlichungstag und Bekanntmachung des Hinweises auf die Patenterteilung: **11.08.2004**
(21) Anmeldenummer: 01123191.7
(22) Anmeldetag: 28.09.2001
(51) Int. Cl.: B65B 19/28, B65B 57/00, G01N 3/08

(54) **Verfahren und Vorrichtung zum Prüfen von insbesondere Zigarettenpackungen**
Method and device for checking cigarette packages
Procédé et dispositif de contrôle d'emballages des cigarettes

(30) Priorität: 10.10.2000 DE 10050297
(43) Veröffentlichungstag der Anmeldung: 17.04.2002
(73) Patentinhaber: Focke & Co. (GmbH & Co.), 27283 Verden (DE)
(72) Erfinder: Focke, Heinz, 27283 Verden (DE); Stiller, Martin, 27283 Verden (DE); Schmidt, Jens, 28879 Grasberg (DE); Sgodzai, Ralph, 27721 Ritterhude (DE); Buse, Henry, 27374 Visselhövede (DE)
(74) Vertreter: Bolte, Erich, Dipl.-Ing.

(56) Entgegenhaltungen:
- EP-A- 0 195 173
- EP-A- 0 360 693
- DE-A- 19 614 475
- US-A- 4 916 883
- US-A- 4 984 409

## Beschreibung

Die Erfindung betrifft Verfahren zum Prüfen von quaderförmigen Zigarettenpackungen aus verformbarem Verpackungsmaterial, wobei die Zigarettenpackung mit definiertem Druck beaufschlagt wird und dadurch bedingte Verformungen der Packung gemessen werden, wobei zur Druckbeaufschlagung das Druckorgan in gleichförmiger Bewegung auf die Zigarettenpackung abgesenkt wird. Weiterhin betrifft die Erfindung eine Vorrichtung zur Durchführung des Verfahrens.

Aus der US 4,984,409 ist ein Verfahren zur Prüfung der Verklebung von Packungen bekannt geworden. Auf die Packungen wird ein Probedruck ausgeübt. Wenn sich fehlerhafterweise ein Klebestreifen von der Verpackung löst, wird dieses Lösen über eine Lichtschranke detektiert.

Die US 4,916,883 offenbart ein Verfahren zur Prüfung der Unversehrtheit des Inhalts einer Weichverpackung für Lebensmittel. Über Fühler wird ein Probedruck auf die Seiten der Weichpackung ausgeübt. Falls der Inhalt, beispielsweise Kekse, fehlerhaft, d.h. zerbrochen ist, dringen die Fühler entsprechend tief in die Packung ein, was über Lichtschranken detektiert wird.

Die Prüfung von Packungen ist besonders wichtig bei der Fertigung von Zigarettenpackungen, für die eine hohe Genauigkeit gefordert wird.

Der Erfindung liegt die Aufgabe zugrunde, Maßnahmen für eine exakte Prüfung von Packungen vorzuschlagen, die darüber hinaus in einfacher Weise durchführbar sind.

Zur Lösung dieser Aufgabe ist das eingangs genannte Verfahren dadurch gekennzeichnet, dass während der Verformung der Packung der zurückgelegte Weg des Druckorgans gemessen wird sowie über ein entsprechendes Messorgan die während des Weges jeweils dem Druckorgan entgegengesetzte Gegenkraft der Packung, dass aus diesen Werten ein Kraft-Weg-Diagramm ermittelt wird, dass dieses Kraft-Weg-Diagramm mit zumindest einem Kraft-Weg-Diagramm gleicher oder gleichartiger Packungen verglichen wird.

Bestandteil der Erfindung ist die Erkenntnis, dass der Verlauf einer durch Druck verursachten Verformung der Packung charakteristisch ist für deren Aufbau und auch für den Inhalt. Des Weiteren können bestimmte Konstruktions- und Gestaltungsmerkmale der Packung anhand des Ablaufs der Verformung erkannt werden.

Der Ablauf der Verformung der Packung wird erfindungsgemäß anhand von Änderungen der für die Verformung aufgewendeten Kraft über einen vorgegebenen Verformungsablauf festgestellt. Konkret wird so vorgegangen, dass ein auf die Packung wirkendes Druckorgan hinsichtlich des zurückgelegten Weges und der während des Verformungsvorgangs auftretenden Kraft gemessen wird. Die Messergebnisse werden fortlaufend aufgezeichnet und ausgewertet, insbesondere durch grafische Darstellung. Diese wird als Ergebnis der Auswertung über einen Rechner als Kurve dargestellt in Abhängigkeit von Kraft und Weg. Das Druckorgan wird dabei vorzugsweise mit gleichförmiger, insbesondere konstanter Bewegung gegen die Packung gedrückt bzw. auf diese abgesenkt. Die aufgrund des Widerstands der Packung sich ergebende Kraft wird gemessen und über den Weg aufgezeichnet.

Eine Besonderheit der Erfindung liegt in der Erkenntnis, dass während des Verformungsvorgangs mehrfach eine Änderung der gemessenen Kraft auftritt, und zwar infolge von Konstruktionsdetails der Verpackung. Bei einem vorteilhaften Anwendungsbeispiel, nämlich bei der Prüfung einer Zigarettenpackung des Typs Klappschachtel, wird während des Verformungsprozesses die aufgewendete Kraft durch Konstruktionsmerkmale wie Deckelinnenlappen, deckelseitige Querkanten und bodenseitige Querkanten erkennbar beeinflusst, so dass die grafische Aufzeichnung des Kraftverlaufs entlang des Verformungsweges ein typisches und wiederholbares Bild über das Verformungsverhalten der Packung gibt.

Die aufgezeichneten und grafisch dargestellten Messergebnisse können erfindungsgemäß einer Vergleichsgrafik bzw. einer gespeicherten "Eichgrafik" gegenübergestellt werden, um etwaige Abweichungen von einer Standardausführung der betreffenden Packung zu erkennen. Eine Besonderheit besteht darin, dass zu diesem Zweck die zweite Ableitung der aus Kraft und Weg gebildeten Kurve ermittelt und aufgezeichnet wird. Die dabei entstehende Kurve weist Kurvenerhöhungen bzw. Peaks auf, die einer erhöhten Kraft bzw. einem erhöhten Widerstand der Packung gegen die Verformung entsprechen und dadurch Rückschlüsse auf die Konstruktion, aber auch auf die Materialzusammensetzung, die Materialstärke und den Packungsinhalt zulassen.

Das vorliegende Mess- bzw. Prüfverfahren ist mit besonderen Vorteilen bei Zigarettenpackungen des Typs Hinge-Lid anwendbar, also bei einer Packung aus dünnem Karton. Diese wird nach Fertigstellung überprüft, und zwar entweder vor oder nach dem Anbringen einer Außenumhüllung aus Folie.

Bei der Umsetzung des Verfahrens wird erfindungsgemäß so vorgegangen, dass die Packung als ganzes zwischen relativ zueinander bewegbaren Druckorganen eingespannt wird, nämlich insbesondere zwischen einer (oberen) Druckplatte und einer (unteren) plattenförmigen Wägezelle. Mindestens ein Organ ist bewegbar. Der zurückgelegte Weg wird gemessen und ebenso die auftretende Kraft. Hieraus ergibt sich das charakteristische Verformungsverhalten der Packung.

Verfahren und Vorrichtung sind in einen Verpackungsprozess integrierbar, vorzugsweise durch Positionierung einer Prüfstation im Bereich eines Packungsförderers, wobei stichprobenartig einzelne Packungen aus dem Förderprozess herausgenommen und hinsichtlich korrekter Ausbildung überprüft werden.

Weitere Besonderheiten der Erfindung werden nachfolgend anhand eines Ausführungsbeispiels einer Vorrichtung und anhand des Auswertungsverfahrens näher erläutert. Es zeigt:
- Fig. 1: eine Vorrichtung in Seitenansicht zur Prüfung einer Packung,
- Fig. 2 bis Fig. 4: grafische Darstellungen über die Verformung einer Packung,
- Fig. 5: eine Maschineneinheit für die Herstellung von Zigarettenpackungen, in schematischem Grundriss,

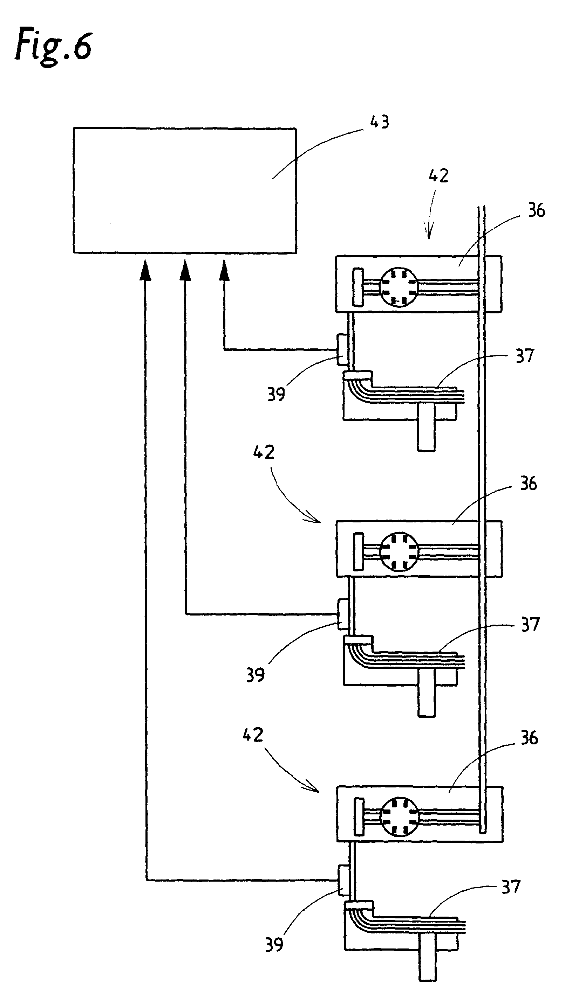

- Fig. 6: eine Anlage aus mehreren Maschineneinheiten, ebenfalls in schematischem Grundriss.

Die nachfolgend beschriebenen Einzelheiten beziehen sich auf das bevorzugte Gebiet der Prüfung von Packungen 10, nämlich von Zigarettenpackungen, und zwar des Typs Hinge-Lid bzw. Klappschachtel. Dieser Typ von Packungen 10 besteht aus dünnem Karton. Die Packung besteht aus einem (unteren) Schachtelteil 11 und einem mit diesem schwenkbar verbundenen Deckel 12. Die Packung 10 ist quaderförmig ausgebildet mit einer großflächigen Vorderseite 13 und entsprechender Rückseite 14. Schachtelteil 11 und Deckel 12 bilden langgestreckte, doppellagige Seitenwände 15. Der Zuschnitt für die Packung 10 ist so gefaltet, dass die Packungsseiten bzw. Wände durch (aufrechte) rechtwinklige Packungskanten 16, 17 und Querkanten 18, 19 begrenzt sind.

Die Packung 10 wird mit Hilfe von Druck bzw. Zusammenpressen in mehrfacher Hinsicht überprüft. Zu diesem Zweck wird die Packung 10 zwischen Druckorganen positioniert, die im Bereich großflächiger Packungsseiten, nämlich im Bereich von Vorderseite 13 und Rückseite 14, an der quaderförmigen Packung 10 anliegen. Durch (Abwärts-)Bewegung mindestens eines Druckorgans wird der Druck auf die Packung 10 übertragen und deren Verformung bewirkt. Hieraus werden Merkmale über Material und Gestaltung der Packung 10 abgeleitet.

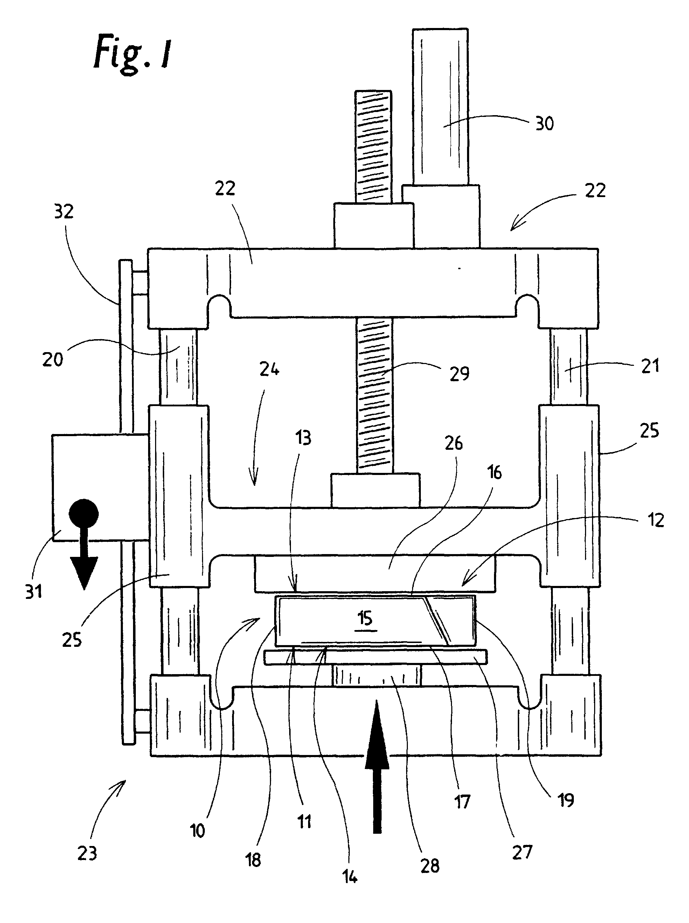

Die in Fig. 1 gezeigte Prüfvorrichtung besteht aus (aufrechten) Tragsäulen 20, 21. Diese sind an den (oberen und unteren) Enden durch quergerichtete Traversen 22, 23 zu einem rahmenförmigen Tragwerk miteinander verbunden. Zwischen den Traversen 22, 23 ist ein Druckorgan verschiebbar, nämlich eine Druckstrebe 24, die mit Gleithülsen 25 auf den Tragsäulen 20, 21 verschiebbar ist. Die Druckstrebe 24 ist Träger für ein Druckorgan, nämlich für eine Druckplatte 26. Diese liegt zur Übertragung von Druck an der Oberseite bzw. an der Vorderseite 13 der Packung 10 an.

Als Gegendruckstück dient eine Tragplatte 27 unterhalb der Packung 10. Diese Tragplatte 27 ist mit einem Druckmessorgan verbunden, und zwar im vorliegenden Falle mit einer Wägezelle 28. Es handelt sich dabei um ein handelsübliches Organ, welches den übertragenen Druck messen und in elektrische Signale umsetzen kann, und zwar insbesondere durch einen Dehnmessstreifen. Durch die Wägezelle 28 wird demnach die auf die Packung 10 ausgeübte Kraft während der gleichförmigen, insbesondere kontinuierlichen Abwärtsbewegung der Druckplatte 26 gemessen.

Die Druckorgane, nämlich Druckplatte 26 und Tragplatte 27 weisen eine Anlagefläche für die Packung 10 auf, die größer ist als diese bzw. als Vorderseite 13 und Rückseite 14. Die Packung 10 wird demnach vollflächig durch die Druckorgane beaufschlagt.

Die Vorrichtung ist so ausgebildet, dass die (untere) Tragplatte 27 feststehend und die Druckplatte 26 zur Übertragung von Druck auf die Packung 10 absenkbar ist. Hierzu ist die Druckstrebe 24 durch ein Getriebe heb- und senkbar, im vorliegenden Falle durch einen Spindeltrieb mit einer aufrechten, in der oberen Traverse drehbar gelagerten Spindel 29. Eine drehbare Spindelmutter wird motorisch angetrieben, nämlich durch einen Elektromotor 30. Durch den (Spindel-)Trieb wird demnach die Druckplatte 26 in einer vorgegebenen, insbesondere kontinuierlichen Förderbewegung gegen die Packung 10 bewegt, und zwar über die Druckstrebe 24. Dabei erzeugt die Packung während des Verformens bzw. Zusammendrückens einen Widerstand, der über die Tragplatte 27 bzw. die Wägezelle 28 gemessen wird.

Die Bewegung des Druckorgans bzw. der Druckplatte 26 einerseits und die dabei auftretende Kraft bei der Verformung der Packung 10 andererseits werden gemessen und aufgezeichnet. Zu diesem Zweck ist der Druckplatte 26 bzw. der Druckstrebe 24 ein Wegsensor 31 bekannter Bauart zugeordnet. Der Wegsensor 31 ist an einer seitlich an der Vorrichtung angebrachten, aufrechten Führungsschiene 32 auf- und abbewegbar, wobei die Wegstrecke exakt gemessen und in elektrische Signale umgesetzt wird.

Eine Besonderheit besteht darin, dass die gemessenen Daten Weg und Kraft grafisch dargestellt werden. Die Daten werden über einen Rechner verarbeitet und als Grafik, nämlich als Kurve aufgezeichnet. Auf der Ordinate wird die gemessene Kraft und auf der Abszisse zurückgelegte Weg aufgezeichnet.

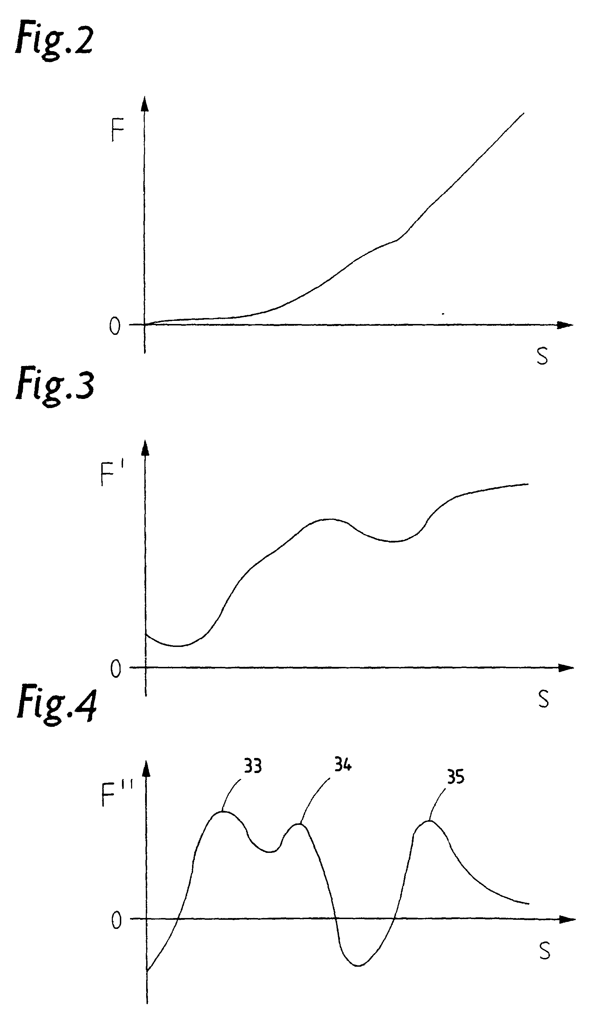

Fig. 2 zeigt die Kurve aufgrund unmittelbarer Wechselbeziehung zwischen Kraft F und Weg S. Die Kurve beginnt bei Berührung der Packung 10 durch die Tragplatte 27 und endet bis zu einer Verformung der Packung ohne Zerstörung derselben, insbesondere bis zum Beginn des Zusammendrückens der Zigaretten.

Fig. 3 zeigt eine Kurve, die als erste Ableitung aufgrund der Kurve gemäß Fig. 2 errechnet ist. Eine günstige Darstellung der Packung 10 hinsichtlich des Verhaltens beim Zusammendrückens ergibt sich aber aus Fig. 4, nämlich der zweiten Ableitung der geschilderten Wechselbeziehung. Die Kurve weist mehrere Kurvenerhöhungen 33, 34, 35 auf, nämlich Peaks, die jeweils für einen durch die Konstruktion der Packung 10 bedingten Druckanstieg stehen, nämlich für einen veränderten bzw. erhöhten Druckwiderstand durch die Packung 10 bei konstanter Abwärtsbewegung des Druckorgans.

Die Kurve gemäß Fig. 4 zeigt Eigenschaften einer Packung 10 des Typs Klappschachtel (Fig. 1). Die Kurve beginnt beim Aufsetzen der Druckplatte 26 auf die Oberseite, nämlich Vorderseite 13 der Packung 10. Die erste Kurvenerhöhung 33 ist zurückzuführen auf eine Krafterhöhung durch einen bei Klappschachteln üblichen Deckel-Innenlappen, also durch eine Materialverstärkung im Bereich der nach oben weisenden Deckel-Vorderwand.

Die folgende Kurvenerhöhung 34 ist auf eine weitere Widerstandserhöhung bei der Verformung der Packung 10 und einen dadurch bedingten Kraftanstieg zurückzuführen, nämlich auf eine erhöhte Steifigkeit im Bereich des Deckels 12. Die end- bzw. eckseitigen Querkanten 19 erhöhen den Widerstand der Packung 10 und verursachen dadurch eine erhöhte Verformungskraft.

Im Anschluss daran, nämlich bei weiterer Druckbewegung der Druckplatte 26, ergibt sich ein erneuter Peak bzw. die Kurvenerhöhung 35 durch eine entsprechende Reaktion der Packung 10. Dieser Kraftanstieg ist durch die Steifigkeit der Packung infolge der bodenseitigen Querkanten 18 bedingt. Unmittelbar danach wird der Vorgang des Zusammendrückens der Packung 10 beendet. Es entsteht dadurch insgesamt eine Kurve gemäß Fig. 4, die charakteristisch ist für einen bestimmten Typ einer Packung, im vorliegenden Falle für eine Klappschachtel.

Eine weitere Besonderheit besteht nun darin, das Verfahren in verschiedener Weise einzusetzen, und zwar zur Identifizierung korrekter oder fehlerhafter Packungen. Dabei wird die Erkenntnis berücksichtigt, dass die Materialstärke, zum Beispiel die Dicke des Kartons, Art und Beschaffenheit einer Innenumhüllung (Innerliner), Art, Beschaffenheit und Formation der Zigarettengruppe sowie weitere Gestaltungsmerkmale der Packung Einfluss auf die sich beim Prüfvorgang unter Druckaufwendung ergebende Kurve haben.

Bei der Prüfung von Packungen wird dabei insbesondere so vorgegangen, dass die durch die Prüfung aufgezeichnete Kurve, insbesondere in der Ausführung der zweiten Ableitung, einer oder mehreren gespeicherten Vergleichskurven gegenübergestellt werden. Dabei kann es sich um eine "Eichkurve" für korrekte, standardgemäße Packungen des herzustellenden Typs handeln. Fehlerhaft ausgebildete Packungen können mit Hilfe dieses Prüfverfahrens identifiziert werden.

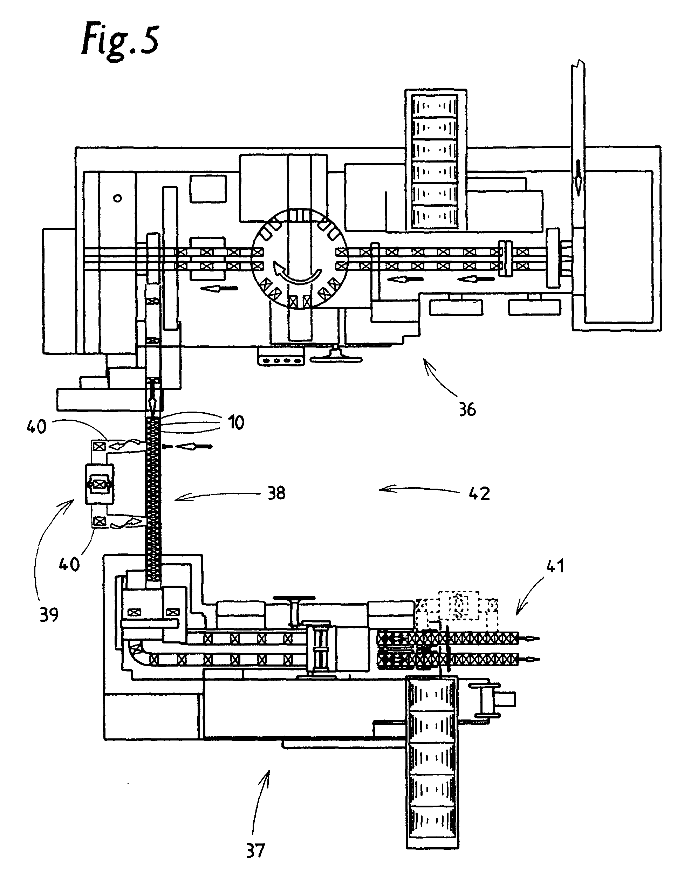

Eine weitere Besonderheit besteht darin, das Prüfverfahren bzw. eine Prüfvorrichtung, zum Beispiel im Sinne von Fig. 1, in den Fertigungsablauf der Packungen 10 zu integrieren. Fig. 5 zeigt das Layout einer Verpackungseinheit für Klappschachteln. Die Einheit besteht aus einer Verpackungsmaschine bzw. einem Packer 36 und einer Verpackungsmaschine für die Herstellung einer Außenumhüllung aus Folie, also einem sogenannten Cello-Packer 37. Beide Verpackungsmaschinen sind durch einen geradlinigen Packungsförderer 38 miteinander verbunden. Im Bereich dieser Förderstrecke ist eine Prüfstation 39 angeordnet. Einzelne Packungen 10 werden stichprobenartig aus dem Bereich des Packungsförderers 38 herausbewegt und über einen Zwischenförderer 40 durch die Prüfvorrichtung gemäß Fig. 1 hindurchgefördert. Die geprüfte Packung wird durch den Zwischenförderer 40 dem Packungsförderer 38 wieder zugeführt. Es ist sinnvoll, die Prüfung der Packungen 10 taktweise durchzuführen. Zusätzlich oder alternativ kann die Prüfstation 39 auch im Anschluss an den Cello-Packer 37 positioniert sein, zum Beispiel im Bereich von an den Cello-Packer 37 anschließenden Packungsbahnen 41.

Die Prüfung der Packungen 10 kann für eine komplette Anlage durchgeführt werden, die aus mehreren Verpackungseinheiten gemäß Fig. 5 bestehen. Die Prüfstationen 39 sind jeweils mit einem einzelnen Rechner und vorzugsweise einem Bildschirm zum Darstellen der Kurven gemäß Fig. 2 bis Fig. 4 vorgesehen. Zusätzlich oder alternativ sind die Prüfstationen 39 der Verpackungseinheiten 42 mit einem zentralen Rechner 43 verbunden, der eine Erfassung der Betriebsdaten durchführt (BDE). Auf diese Weise sind Informationen und Erkenntnisse einer gesamten Verpackungsanlage zentral erfassbar.

Das Prüfverfahren lässt Rückschlüsse zu über etwaige Fehlerursachen. Diese können in der Packung selbst bzw. im Verpackungsmaterial, aber auch in einem Verhalten der Verpackungsmaschine liegen. Die Charakteristik der gemessenen Werte bzw. der aufgezeichneten Kurve lässt Rückschlüsse hierüber zu. Des Weiteren ist es möglich, anhand einer bei der Prüfung festgestellten allmählichen Veränderung im Verhalten der Packungen Rückschlüsse auf einen Maschinenverschleiß zu ziehen.

### Bezugszeichenliste:

- 10: Packung
- 11: Schachtelteil
- 12: Deckel
- 13: Vorderseite
- 14: Rückseite
- 15: Seitenwand
- 16: Packungskante
- 17: Packungskante
- 18: Querkante
- 19: Querkante
- 20: Tragsäule
- 21: Tragsäule
- 22: Traverse
- 23: Traverse
- 24: Druckstrebe
- 25: Gleithülse
- 26: Druckplatte
- 27: Tragplatte
- 28: Wägezelle
- 29: Spindel
- 30: Elektromotor
- 31: Wegsensor
- 32: Führungsschiene
- 33: Kurvenerhöhung
- 34: Kurvenerhöhung
- 35: Kurvenerhöhung
- 36: Packer
- 37: Cello-Packer
- 38: Packungsförderer
- 39: Prüfstation
- 40: Zwischenförderer
- 41: Packungsbahn
- 42: Verpackungseinheit
- 43: zentraler Rechner

## Patentansprüche

1. Verfahren zum Prüfen von quaderförmigen Zigarettenpackungen (10) aus verformbarem Verpackungsmaterial, wobei die Zigarettenpackung (10) mit definiertem Druck beaufschlagt wird und dadurch bedingte Verformungen der Packung (10) gemessen werden, wobei zur Druckbeaufschlagung das Druckorgan (26) in gleichförmiger Bewegung auf die Zigarettenpackung (10) abgesenkt wird, **dadurch gekennzeichnet, dass**
a) während der Verformung der Packung (10) der zurückgelegte Weg des Druckorgans (26) gemessen wird sowie über ein entsprechendes Messorgan (28) die während des Weges jeweils dem Druckorgan (26) entgegengesetzte Gegenkraft der Packung (10),
b) dass aus diesen Werten ein Kraft-Weg-Diagramm ermittelt wird,
c) dass dieses Kraft-Weg-Diagramm mit zumindest einem Kraft-Weg-Diagramm gleicher oder gleichartiger Packungen verglichen wird.

2. Verfahren nach 1, **dadurch gekennzeichnet, dass** der Weg über einen Wegsensor (31) gemessen und dass die Kraft über eine Wägezelle (28) gemessen wird.

3. Verfahren nach Anspruch 1 oder 2, **dadurch gekennzeichnet, dass** der sich bei zunehmender Verformung der Packung (10) aufgrund der gleichförmigen Bewegung des Druckorgans verändernde Widerstand der Packung (10) (Gegenkraft) grafisch angezeigt wird.

4. Verfahren nach Anspruch 3, **dadurch gekennzeichnet, dass** die grafische Darstellung des Verlaufs der auf die Packung (10) wirkenden Kraft bei der Verformung derselben als Kurve dargestellt wird.

5. Verfahren nach Anspruch 2 oder einem der weiteren vorhergehenden Ansprüche, **dadurch gekennzeichnet, dass** der Verlauf der auf die Packung (10) wirkenden Kraft bei gleichförmiger Bewegung des Druckorgans in einer Ableitung, insbesondere als zweite Ableitung, dargestellt wird.

6. Verfahren nach Anspruch 1 oder einem der weiteren vorhergehenden Ansprüche, **dadurch gekennzeichnet, dass** die Kraft flächig auf die Packung (10) übertragen wird, insbesondere über eine volle Packungsfläche, vorzugsweise auf der gesamten großflächigen Vorderseite (13) oder Rückseite (14) einer quaderförmigen Packung.

7. Verfahren nach Anspruch 1 oder einem der weiteren vorhergehenden Ansprüche, **dadurch gekennzeichnet, dass** das Druckorgan bei konstanter Bewegung auf die Packung (10) aufgebracht und die im Bereich der Packung (10) wirkende Kraft durch ein Kraftmessorgan festgestellt wird.

8. Verfahren nach Anspruch 2, **dadurch gekennzeichnet, dass** die Messergebnisse der Wägezelle (28) einerseits und des Wegsensors (31) andererseits in einem Rechner ausgewertet und anhand einer Kurve, vorzugsweise in der Ausführung als zweiter Ableitung, dargestellt werden.

9. Vorrichtung zum Prüfen von Zigarettenpackungen (10) aus verformbarem Verpackungsmaterial, wobei die Zigarettenpackung (10) zwischen einander gegenüberliegenden Druckorganen (26, 27) positioniert ist, wobei mindestens ein Druckorgan (26) gegen die Packung (10) bewegbar ist, **dadurch gekennzeichnet, dass** an dem gegen die Packung (10) bewegbaren Druckorgan (26) ein Wegmessorgan (31) und an dem anderen Druckorgan (27) eine Wägezelle (28) angeordnet ist.

10. Vorrichtung nach Anspruch 9, **dadurch gekennzeichnet, dass** die Druckorgane (26, 27) eine obere Druckplatte (26) und eine untere Tragplatte (27) aufweisen, wobei die Druckplatte (26) an einem auf- und abbewegbaren Träger angebracht ist, insbesondere an einer Druckstrebe (24), die durch ein gleichförmig angetriebenes Getriebe bewegbar ist.

11. Vorrichtung nach Anspruch 10, **gekennzeichnet durch** ein Traggestell mit einer oberen Traverse (22) und einer unteren Traverse (23), die **durch** Tragsäulen (20, 21) miteinander verbunden sind, wobei die Druckstrebe (24) auf den Tragsäulen (20, 21) verschiebbar gelagert und die Wägezelle (28) auf der unteren Traverse (23) positioniert ist.

12. Vorrichtung nach Anspruch 10 oder 11, **dadurch gekennzeichnet, dass** an der bewegbaren Druckstrebe (24) ein Wegsensor (31) angebracht ist.

13. Vorrichtung nach Anspruch 9 oder einem der weiteren vorhergehenden Ansprüche, **dadurch gekennzeichnet, dass** eine Prüfstation (39) mit einer Vorrichtung zur Druckverformung einer Packung (10) einer Verpackungseinheit (42) zugeordnet ist zur vorzugsweise stichprobenartigen Prüfung von Packungen (10).

14. Vorrichtung nach Anspruch 13, **dadurch gekennzeichnet, dass** die Prüfstation im Bereich eines Packungsförderers (38) zwischen einem Packer (36) und einem Cello-Packer (37) positioniert ist.

15. Vorrichtung nach Anspruch 13 oder einem der weiteren Ansprüche, **dadurch gekennzeichnet, dass** mehrere Verpackungseinheiten (42) mit mindestens je einer Prüfstation (39) hinsichtlich des Prüfergebnisses mit einem zentralen Rechner (43) zum zentralen Erfassen der Betriebsdaten verbunden sind.

## Claims

1. Method for the testing of cuboid-shaped packs (10) of formable packaging material, the cigarette pack (10) being impinged with a defined pressure and the resulting deformations of the pack (10) being measured, it being the case that during the application of pressure, the pressure-exerting means (26) is lowered onto the cigarette pack (10) in a uniform motion, **characterized by** the following features:
a) during the deformation of the pack (10) continuous measurement is made of the distance covered by the pressure-exerting means (26) as well as of the counterforce of the pack (10) exerted in each case against the pressure-exerting means (26) and determined by a corresponding measuring device (28),
b) a force/distance diagram is determined from the measured values,
c) this force/distance diagram is compared with at least one force/distance diagram of the same or similar packs.

2. Method according to Claim 1, **characterized in that** the distance is measured by means of a position sensor (31) and the force is measured by means of a load cell (28).

3. Method according to Claim 1 or 2, **characterized in that** the resistance of the pack (10) as counterforce, which varies as the result of increasing deformation of the pack (10) under uniform movement of a pressure-exerting means, is displayed in graphic form.

4. Method according to Claim 3, **characterized in that** the graphic representation of the course of force acting on the pack (10) during its deformation is plotted as a curve.

5. Method according to Claim 2 or one of the further preceding Claims, **characterized in that** the course of force acting on the pack (10) during uniform movement of the pressure-exerting means is represented as a derivative, in particular as a second derivative.

6. Method according to Claim 1 or one of the further preceding Claims, **characterized in that** force is transferred to the pack surface (10), in particular across the entire pack surface, preferably on the entire large-surface front side (13) or rear side (14) of a cuboid-shaped pack.

7. Method according to Claim 1 or one of the further preceding Claims, **characterized in that** the pressure-exerting means is applied to the pack (10) at a constant movement and that the force acting in the region of the pack (10) is measured by a pressure gauge.

8. Method according to Claim 2, **characterized in that** the measuring results of the load cell (28), on one hand, and those of the position sensor (31) are evaluated by a computer and plotted as a curve, preferably in the form of its second derivative.

9. Apparatus for the testing of cigarette packs (10) of formable packaging material, with the cigarette pack (10) being positioned between opposing pressure-exerting means (26, 27), it being possible to move at least one pressure-exerting means (26) against the pack (10), **characterized in that** a position sensor (31) is arranged on the pressure-exerting means (26) moved against the pack (10) and that a load cell (28) is arranged on the other pressure-exerting means (27).

10. Apparatus according to Claim 9, **characterized in that** the pressure-exerting means (26, 27) have an upper pressure plate (26) and a lower bearing plate (27), the pressure plate (26) being mounted on a carrier that can be moved up and down, in particular on a pressure strut (24), which can be displaced by means of a uniformly driven gear mechanism.

11. Apparatus according to Claim 10, **characterized by** a supporting framework with an upper traverse (22) and a lower traverse (23), which are connected to one another by supporting columns (20, 21), with the pressure strut (24) being displaceably mounted on the supporting columns (20, 21) and the load cell (28) positioned on the lower traverse (23).

12. Apparatus according to Claim 10 or 11, **characterized in that** a position sensor (31) is attached to the displaceable pressure strut (24).

13. Apparatus according to Claim 9 one of the further preceding Claims, **characterized in that** a test station with a device for the deformation by compression of a pack is assigned a packaging unit (42) for the preferably random testing of packs.

14. Apparatus according to Claim 13, **characterized in that** the test station (39) is positioned in the region of a pack conveyor (38) between a packer (36) and a cello-packer (37).

15. Apparatus according to Claim 13 or one of the further preceding Claims, **characterized in that** a plurality of packaging units (42) having at least one test station (39) each are connected to a central computer (43) for the central logging of operational data concerning the testing results

## Revendications

1. Procédé de contrôle de paquets de cigarettes parallélépipédiques rectangles (10) en matériau d'emballage déformable, dans lequel une pression définie est appliquée au paquet de cigarettes (10) et les déformations ainsi produites du paquet (10) sont mesurées, et pour l'application de la pression, l'organe de pression (26) est descendu d'un mouvement uniforme sur le paquet de cigarettes (10), **caractérisé par le fait que**
a) pendant la déformation du paquet (10) sont mesurés le déplacement de l'organe de pression (26) et, par un organe de mesure correspondant (28), la force antagoniste du paquet (10) opposée à l'organe de pression (26) pendant le déplacement,
b) à partir de ces valeurs est établi un diagramme force-déplacement,
c) ce diagramme force-déplacement est comparé à au moins un diagramme force-déplacement de paquets identiques ou semblables.

2. Procédé selon la revendication 1, **caractérisé par le fait que** le déplacement est mesuré au moyen d'un capteur de déplacements (31) et la force est mesurée au moyen d'un peson (28).

3. Procédé selon l'une des revendications 1 et 2, **caractérisé par le fait que** la résistance du paquet (10) (force antagoniste), qui varie lorsque la déformation du paquet (10) croît en raison du mouvement uniforme de l'organe de pression, est indiquée graphiquement.

4. Procédé selon la revendication 3, **caractérisé par le fait que** la représentation graphique de la variation de la force qui agit sur le paquet (10) lors de la déformation de celui-ci est faite sous forme de courbe.

5. Procédé selon la revendication 2 ou une des autres revendications précédentes, **caractérisé par le fait que** la variation de la force qui agit sur le paquet (10) lors du mouvement uniforme de l'organe de pression est représentée sous forme de dérivée, en particulier de dérivée seconde.

6. Procédé selon la revendication 1 ou une des autres revendications précédentes, **caractérisé par le fait que** la force est transmise au paquet (10) sur une surface, en particulier sur une surface entière de celui-ci, de préférence sur toute la face avant (13) ou la face arrière (14) de grande surface d'un paquet parallélépipédique rectangle.

7. Procédé selon la revendication 1 ou une des autres revendications précédentes, **caractérisé par le fait que** l'organe de pression est appliqué sur le paquet (10) avec un mouvement constant, et la force qui agit dans la zone du paquet (10) est déterminée par un organe dynamométrique.

8. Procédé selon la revendication 2, **caractérisé par le fait que** les résultats de mesure d'une part du peson (28) et d'autre part du capteur de déplacements (31) sont exploités dans un calculateur et représentés à l'aide d'une courbe, de préférence sous forme de dérivée seconde.

9. Dispositif de contrôle de paquets de cigarettes (10) en matériau d'emballage déformable, dans lequel le paquet de cigarettes (10) est placé entre des organes de pression opposés (26, 27), au moins un organe de pression (26) étant mobile vers le paquet (10), **caractérisé par le fait que** sur l'organe de pression (26) mobile vers le paquet (10) est monté un organe de mesure de déplacements (31) et sur l'autre organe de pression (27) est monté un peson (28).

10. Dispositif selon la revendication 9, **caractérisé par le fait que** les organes de pression (26, 27) présentent une plaque supérieure de pression (26) et une plaque support inférieure (27), le plaque de pression (26) étant montée sur un support pouvant monter et descendre, en particulier sur une entretoise de pression (24) qui est mue par un mécanisme à mouvement uniforme.

11. Dispositif selon la revendication 10, **caractérisé par** un bâti support comprenant une traverse supérieure (22) et une traverse inférieure (23) qui sont reliées par des colonnes supports (20, 21), l'entretoise de pression (24) étant montée mobile sur les colonnes supports (20, 21) et le peson (28) étant placé sur la traverse inférieure (23).

12. Dispositif selon l'une des revendications 10 et 11, **caractérisé par le fait qu'**un capteur de déplacements (31) est monté sur l'entretoise mobile de pression (24).

13. Dispositif selon la revendication 9 ou une des autres revendications précédentes, **caractérisé par le fait qu'**un poste de contrôle (39) comportant un dispositif de déformation d'un paquet (10) par pression est associé à une unité d'empaquetage pour le contrôle des paquets (10) de préférence par échantillonnage.

14. Dispositif selon la revendication 13, **caractérisé par le fait que** le poste de contrôle est placé dans la zone d'un transporteur de paquets (38) entre une empaqueteuse (36) et une cellophaneuse (37).

15. Dispositif selon la revendication 13 ou une des autres revendications, **caractérisé par le fait que** plusieurs unités d'empaquetage (42) ayant chacune au moins un poste de contrôle (39) sont reliées en ce qui concerne les résultats du contrôle à un calculateur central (43) pour la saisie centrale des données d'exploitation.
